# EUROPEAN PATENT APPLICATION

(11) **EP 4 473 982 A1**
(43) Date of publication of application: **11.12.2024**
(21) Application number: 24177939.6
(22) Date of filing: 24.05.2024
(51) Int. Cl.: A61L 9/12

(54) **AIR FRESHENING DEVICE WITH ROD**

(30) Priority: 25.05.2023 ES 202330909 U
(71) Applicant: ZENIT Estudio de Diseño e Innovación S.L., 46010 Valencia (ES)
(72) Inventor: BLASCO FEO, Vicente, 46010 Valencia (ES)
(74) Representative: Soler Lerma, Santiago

(57) **Abstract**

The present invention consist of an air freshening device with rod, comprising an impregnated element (6) housed in a box (5) closed by a lid (7) characterised in that it comprises a rod (1) with a clamp (2) and a free end (3) suitable for insertion into a housing (4) of the box (5) and characterised in that the impregnated element (6) has a fixing hole (11) at its rear facing the housing (4) of the box and that the lid (7) has a window (8)

## Description

The invention, as its name indicates, is an air freshening device with a fastening element suitable, preferably, for being fixed to the ventilation grille of a vehicle, where its characteristics allow a greater emanation of the air freshening flow and at the same time maintain the device in optimal air freshening and aesthetic conditions throughout its useful life.

The device comprises a container, a lid that closes the assembly and allows an easy emanation of flow into the atmosphere thanks to a wide opening that links the inside of the container with the outside, a fastening element such as a clamp and an air freshener pad.

The device has the means to prevent the air freshener from moving or even leaving the container so that it loses moisture and reduces in size, despite the large opening in the lid.

### BACKGROUND TO THE INVENTION

At present, air fresheners that have an element impregnated with volatile substances that, by the action of the air, release the fragrance impregnated in its structure, are well known.

Within the state of the art of these air fresheners, we can find several that are used by placing them next to a stream of air, such as in the path of the air coming from a ventilation grille.

Patent KR20170031579 relates to a device that presents a blister type replacement with a plurality of cavities, in each of which is housed a tablet, preferably spherical, of material impregnated in such a way that the user, pressing the cavities of the blister and breaking the seal activates the number of tablets desired. The blister is arranged in a support that is installed in the air stream of the car's ventilation system.

Patent EP1992365 relates to a housing comprising a rear and a front part, suitable for arranging a reservoir with perfume therein and wherein the parts of that housing can rotate relative to each other by varying the air flow through the device.

In general terms, this type of device has a large amount of plastics in its composition which makes them unviable from an environmental point of view.

In this type of device, it is normal for the user to have to manipulate the device, causing the contents of the container to spill.

None of these devices discloses an air freshener that comprises a ventilated container with the means for fastening it to, for example, the ventilation grille of a vehicle where said air freshener has a fastening element that holds the air freshener tablet throughout its useful life, preventing it from moving inside the container or even from coming out of said container as its size decreases due to use.

### DESCRIPTION OF THE INVENTION

The invention relates to a reusable air freshening device with a holding element comprising a ventilated container which houses an element impregnated with volatile substances.

The device comprises:
- An impregnated element, such as an impregnated tablet
- A rod comprising:
   - At one end, a fastening element of the assembly, such as a suitable clamp to be attached to a rack.
   - At the opposite end, an extension suitable for fixing the rod to a box and, in one possible embodiment, fixing the impregnated element in turn.
- A container which in turn comprises:
   - A box with a rod extension housing suitable for the rod extension to be inserted.
   - A lid with a preferably wide window.
   - Means of attachment between the lid and the box and optionally a weatherstrip that can be adjusted.

The rod has, as already mentioned, a clamp at one of its ends and, at the opposite end, an extension suitable for insertion into the housing that the box has at the back. In a preferred embodiment, said housing comprises a through hole that allows the end of the housed rod to protrude inside the box and insert its tip into the impregnated element in such a way that this impregnated element is fixed to the box by the action of the end of the rod.

In another possible embodiment, the housing comprises a countersunk recess with an internal hole wherein said recess projects towards the inside of the box, wherein the impregnated element is fixed to said recess and the free end of the rod is inserted into said hole.

In a possible embodiment, the rod extension has a thread compatible with the internal geometry of the housing.

The term clamp should be understood as referring to any geometry with extensions, wherein said extensions are suitable for being fixed to a body where said body is arranged between them, whether or not pressure is applied.

In a preferred embodiment the impregnated element is attached to the box through a fixing hole that presents said impregnated element facing the housing in such a way that either the tip of the rod or the countersunk recess is inserted into that hole, thereby fixing the impregnated element. This fixing hole can vary its size to adapt it to the element to be inserted.

The impregnated element preferably has the shape of a circular tablet of a size suitable to be housed inside the box that can also have a circular shape or any other shape that allows the impregnated element to be properly housed.

The lid has at least one window that links the inside of the box with the outside, easily allowing the transit of the air flow from the impregnated element to the atmosphere.

As the impregnated pellet expends its useful life, its decreases in size as it loses moisture, but thanks to the fact that the tablet is attached to the rod, it is prevented from coming out of the window of the lid.

The lid is fixed to the box through a common means such as a thread or a clip, although in a possible embodiment for example in wood, the clearances of the materials may make it convenient to use a weatherstrip such as an O-ring.

### BRIEF DESCRIPTION OF THE FIGURES

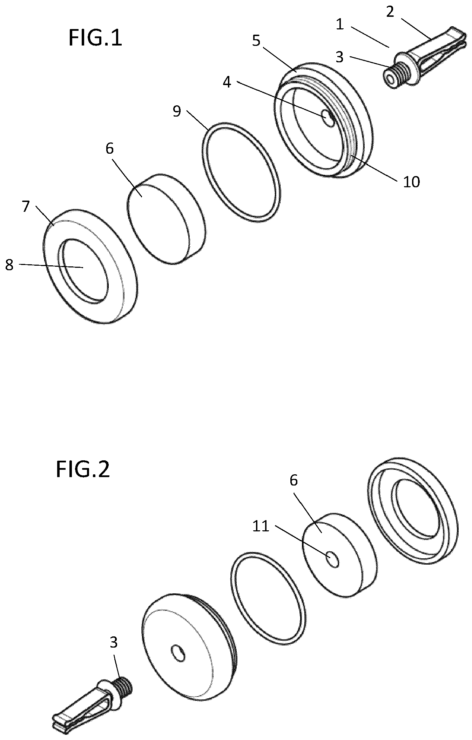

FIGURE 1 shows an exploded view of the device in which the rod (1) is seen with a clamp (2) and a free end (3) which in this case appears with an adjustment relief and which is suitable to be inserted tightly into the housing (4) of the box (5) which in this case is a through hole that will allow the tip of the end of the rod to protrude inside the box that is inserted into the impregnated element (6). The lid (7) with a window (8) and the O-ring (9) that will serve as an adjustment between the lid and the box that, in addition, have joining means, (10) in this case a thread, can also be seen.
FIGURE 2 shows an exploded view of the device in such a way that all the elements revealed in Figure 1 are identified and, in addition, the hole (11) of the impregnated element (6) is suitable for the insertion of the tip of the end (3) of the rod.

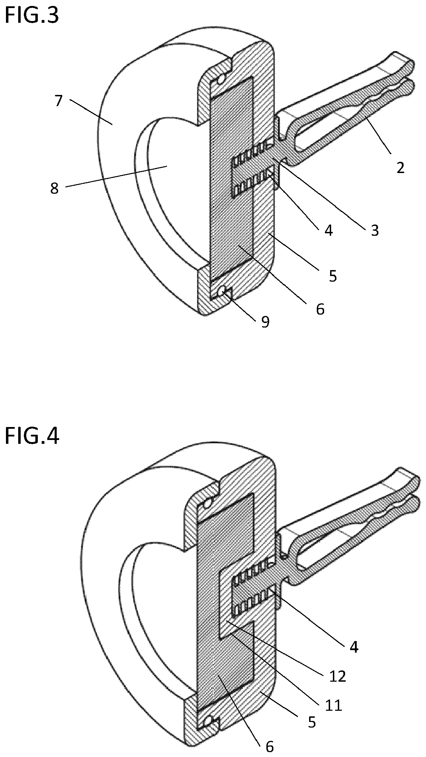

FIGURE 3 shows the device in section, showing the clamp (2), the free end (3) of the rod here inserted in the housing (4) of the box (5) in such a way that the tip of the free end of the rod protrudes inside the box being inserted into the impregnated element (6) which is enclosed by the lid (7) but linked to the outside through the window (8). The O-ring (9) arranged between the lid and the box.
FIGURE 4 shows an alternative embodiment in which the housing (4) suitable for inserting the free end (3) of the rod has a countersunk recess (12) that projects towards the inside of the box (5), said recess being the one that is inserted into the fixing hole (11) of the impregnated element (6) and an internal hole suitable for inserting the free end of the rod.

### DESCRIPTION OF AN EMBODIMENT

A method of implementation is presented here that is not unique or limiting, but merely explanatory.

The device comprises:
A rod (1) suitable for attaching the device to a fixed element such as the ventilation grille of a car, comprising:
a clamp (2) at one end of the rod while the other end (3) is free and has a suitable geometry to be inserted tightly into a housing (4).

A box (5) with a housing (4) arranged on its back. This housing comprises a suitable through hole for the free end (3) of the rod to be inserted into and protrude from the inside of the box.

An impregnated element (6) that is arranged inside the box and that receives the free end (3) of the rod in a hole (11) in its rear face.

A lid (7) that closes the box and has a wide window (8) that links the inside and outside of the box allowing the air flow emanating from the impregnated element to disperse.

The lid and the box are joined by a thread that is removable, although to reinforce that joint an O-ring (9) is provided that saves the possible clearances that, due to the materials, may exist at the joint between the lid and the box.

The box (5) and the lid (7) are made of wood and their geometries facilitate their manufacture by lathe since the box is shaped like a circular plate or tray with a through hole at its central point and the lid is also shaped like a circular plate but with an internal outlet that forms the window (8) and that leaves only a body in the shape of a perimeter ring.

The means that facilitate the link between the box and the lid comprise a screw with a thread and the adjustment O-ring (9).

The rod (1) comprises a clamp at one of its ends, wherein this clamp is suitable for fixing to a laminar part such as the grille of the ventilation system of a car.

The free end (3) of the rod is the one that is fixed to the box by being inserted into the housing (4) and fixing the impregnated element (6).

In this way, this impregnated element is fixed to the box and there is no risk that as it reduces in size as it loses moisture, it can exit through the window (8) falling to the floor, which allows the realization of a window (8) of considerable size that offers a fluid link between the outside and the inside of the box (5) where the impregnated element (6) is housed.

## Claims

1. AIR FRESHENING DEVICE WITH ROD, comprising an impregnated element (6) housed in a box (5) closed by a lid (7) **characterised in that** it comprises a rod (1) with a clamp (2) and a free end (3) suitable for insertion into a housing (4) of the box (5) and **characterised in that** the impregnated element (6) has a fixing hole (11) at its rear facing the housing (4) of the box and that the lid (7) has a window (8).

2. AIR FRESHENING DEVICE WITH ROD according to claim 1, **characterised in that** the housing (4) comprises a through hole suitable for the free end (3) of the rod to be inserted in a tight manner and its tip protrudes from the inside of the box being inserted into the fixing hole (11) of the impregnated element (5).

3. AIR FRESHENING DEVICE WITH ROD according to claim 1, **characterised in that** the housing (4) comprises a countersunk recess (12) with an internal hole suitable for the free end (3) of the rod to be housed therein and it is the recess that is inserted into the fixing hole (11) of the impregnated element.

4. AIR FRESHENING DEVICE WITH ROD according to claim 1, **characterised in that** the housing (4) and the fixing hole (11) of the impregnated element are in a central position.

5. AIR FRESHENING DEVICE WITH ROD according to claim 1, **characterised in that** the box (5) and the lid (7) are circular in shape.

6. AIR FRESHENING DEVICE WITH ROD according to claim 1 **characterised in that** the box and lid are made of wood.
